# DEMANDE DE BREVET EUROPEEN

(11) **EP 0 896 818 A1**
(43) Date de publication de la demande: **17.02.1999**
(21) Numéro de dépôt: 98401858.0
(22) Date de dépôt: 21.07.1998
(51) Int. Cl.: A61K 9/20, A61K 9/00

(54) **Comprimé multiparticulaire perfectionné à délitement rapide**

(30) Priorité: 21.07.1997 FR 9709233
(71) Demandeur: LABORATOIRES PROGRAPHARM, 28170 Châteauneuf-en-Thymerais (FR)
(72) Inventeur: Chauveau, Charles, 06560 Valbonne (FR); Gendrot, Edouard, 28500 Garnay (FR); Demichelis, Alain Gilles, 06130 Grasse (FR); Nouri, Noureddine, 06220 Vallauris (FR)
(74) Mandataire: Koch, Gustave

(57) **Abrégé**

L'invention a pour objet un comprimé multi-particulaire perfectionné à délitement rapide du genre de ceux qui se désintègrent dans la bouche en moins de 40 secondes et qui comprennent, d'une part, une substance active sous forme de microcristaux enrobés par un agent d'enrobage et, d'autre part, un excipient.

## Description

L'invention a pour objet un comprimé multiparticulaire perfectionné à délitement rapide du genre de ceux qui se désintègrent dans la bouche en moins de 40 secondes et qui comprennent, d'une part, une substance active sous forme de microcristaux enrobés par un agent d'enrobage et, d'autre part, un excipient.

La substance active peut être choisie dans le groupe comprenant les antalgiques, les antipyrétiques, les antidiarrhéiques, les antispasmodiques, les régulateurs de la motricité digestive et les anti-inflammatoires et plus particulièrement dans celui comprenant le paracétamol, l'ibuprofène, l'aspirine, le kétoprofène, le lopéramide.

On connaît, par le brevet français FR 91 09245, des comprimés multiparticulaires du genre en question qui donnent en général satisfaction; toutefois, la texture de certains de ces comprimés donne naissance à une sensation sableuse et pâteuse au moment de l'ingestion.

L'invention a pour but, surtout, de remédier à cet inconvénient et de fournir un comprimé à délitement rapide à texture systématiquement agréable, ce comprimé devant de plus conduire à une biodisponibilité optimale du principe actif.

La Société Demanderesse a eu le mérite de trouver, à l'issue de recherches approfondies, que ce but pouvait être atteint, d'une part, en faisant comporter à l'excipient un agent désintégrant ainsi qu'au moins un agent soluble diluant particulier à propriétés liantes et, d'autre part, en sélectionnant l'agent d'enrobage en fonction des caractéristiques physico-chimiques de la substance active.

Il s'ensuit que le comprimé multiparticulaire perfectionné conforme à l'invention, qui se désintègre dans la bouche en moins de 40 secondes et qui comprend d'une part, une substance active sous forme de microcristaux enrobés et, d'autre part, un excipient, est caractérisé par le fait
- que l'excipient comprend au moins un agent désintégrant et au moins un agent soluble diluant à propriétés liantes constitué par un polyol de moins de 13 atomes de carbone se présentant soit sous la forme du produit directement compressible dont le diamètre moyen des particules est de 100 à 500 micromètres, soit sous la forme d'une poudre dont le diamètre moyen des particules est inférieur à 100 micromètres, ce polyol étant de préférence choisi dans le groupe comprenant le mannitol, le xylitol, le sorbitol et le maltitol, étant entendu que le sorbitol ne peut être utilisé seul et que, dans le cas où l'agent soluble diluant à propriétés liantes est unique, il est utilisé sous la forme du produit directement compressible alors que, dans le cas où il y a au moins deux agents solubles diluants à propriétés liantes, l'un est présent sous la forme directement compressible et l'autre sous la forme poudre, le polyol pouvant alors être le même, les proportions de polyol directement compressible et de polyol poudre étant de 99/1 à 50/50, de préférence de 80/20 à 50/50 et
- que l'enrobage des microcristaux de substance active comprend au moins un agent d'enrobage choisi, en fonction des caractéristiques physico-chimiques de la substance active, dans le groupe comprenant les polyméthacrylates, les polymères cellulosiques, notamment les éthylcelluloses, les hydroxypropyl-méthylcelluloses, les hydroxypropyl-celluloses et les acétophthalates de cellulose et les combinaisons de ces polymères l'un avec l'autre éventuellement associés à des plastifiants ou des agents solubles, notamment les polyols.

L'invention pourra être mieux comprise à l'aide du complément de description qui suit et des exemples non limitatifs relatifs à des modes de réalisation avantageux.

Se proposant de préparer un comprimé multiparticulaire du genre en question, on s'y prend comme suit ou de façon équivalente.

On sélectionne tout d'abord la matière active dans le groupe comprenant les antalgiques, les antipyrétiques, les antidiarrhéiques, les antispasmodiques, les régulateurs de la motricité digestive et les anti-inflammatoires et plus particulièrement dans le groupe comprenant le paracétamol, l'ibuprofène, l'aspirine, le kétoprofène, le lopéramide.

On prépare l'excipient en effectuant le mélange d'au moins un agent désintégrant et d'au moins un agent soluble diluant à propriétés liantes constitué par un polyol de moins de 13 atomes de carbone se présentant soit sous la forme du produit directement compressible dont le diamètre moyen des particules est de 100 à 500 micromètres, soit sous la forme d'une poudre dont le diamètre moyen des particules est inférieur à 100 micromètres, ce polyol étant de préférence choisi dans le groupe comprenant le mannitol, le xylitol, le sorbitol et le maltitol, le sorbitol ne pouvant être utilisé seul.

Lorsque l'agent soluble diluant à propriétés liantes est unique, donc différent du sorbitol, il est utilisé sous la forme du produit directement compressible.

Lorsqu'on a recours à au moins deux agents solubles diluants à propriétés liantes, l'un est présent sous la forme du produit directement compressible et l'autre, qui peut être constitué par le même polyol, sous la forme d'une poudre dont le diamètre moyen des particules constitutives est inférieur à 100 micromètres, les proportions de polyol directement compressible et de polyol poudre étant de 99/1 à 50/50, de préférence de 80/20 à 50/50.

L'agent désintégrant est de préférence choisi dans le groupe comprenant la polyvinylpyrrolidone réticulée désignée dans le métier par le terme crospovidone et la carboxyméthylcellulose de sodium réticulée désignée dans le métier par le terme croscarmellose de sodium.

Les proportions respectives d'agent désintégrant et d'agent liant ou diluant soluble retenues pour la constitution de l'excipient sont, par rapport à la masse du comprimé, de 3 à 15% en poids, de préférence de 5 à 10% en poids pour le premier et de 40 à 90% en poids, de préférence de 50 à 70% en poids pour le second; la proportion maximale de sorbitol est de 30% en poids.

Le principe actif entrant dans la constitution du comprimé conforme à l'invention se présente avant enrobage sous forme de microcristaux dont le diamètre moyen est d'environ 1 à environ 500 micromètres.

Dans le cas de certains principes actifs, on utilise de préférence des microcristaux de diamètre moyen inférieur à 100 micromètres afin d'augmenter la surface d'échange entre le principe actif et le milieu ambiant; en procédant ainsi, la vitesse de solubilisation et/ou la solubilité intrinsèque de dissolution sont optimisées.

L'enrobage des microcristaux est réalisé de préférence suivant la technique dite du lit fluidisé.

On procède à un enrobage direct dans le cas des microcristaux dont la taille moyenne est de 100 à 500 micromètres; pour les microcristaux de taille inférieure à 100 micromètres, on procède à un traitement préalable consistant soit en une granulation des microcristaux par un procédé de granulation humide ou sèche classique, soit à une fixation préalable des microcristaux sur des supports neutres en eux-mêmes connus lorsque la taille moyenne desdits microcristaux est inférieure à environ 20 micromètres; la fixation des microcristaux sur les supports neutres est effectuée de manière classique en utilisant un liant constitué par exemple par l'hydroxypropyl-méthylcellulose.

L'enrobage est constitué à partir d'au moins un agent d'enrobage choisi, en tenant compte des caractéristiques physico-chimiques du principe actif, dans le groupe comprenant les polyméthacrylates, notamment certains de ceux commercialisés sous la marque EUDRAGIT et plus particulièrement les dispersions à 30% de poly(éthylacrylate-méthylmétacrylate) commercialisées sous la marque de fabrique EUDRAGIT NE 30 D, les copolymères d'aminoalkyl-méthacrylate de type E commercialisés sous la marque EUDRAGIT E, les polymères cellulosiques, notamment les éthylcelluloses, les hydroxypropyl-méthylcelluloses, les hydroxypropylcelluloses et les acétophthalate de cellulose, les combinaisons de ces polymères l'un avec l'autre éventuellement associés à des plastifiants comme par exemple le polyéthylèneglycol 6000 ou à des agents solubles, notamment des polyols comme par exemple le mannitol.

A titre d'exemple, on signale que l'enrobage peut être constitué à partir
- d'EUDRAGIT NE 30 D seul ou en mélange avec l'EUDRAGIT E dans un ou plusieurs solvants organiques,
- d'éthylcellulose seule ou en mélange avec une hydroxypropyl-méthylcellulose associée à un plastifiant en présence éventuellement d'un solvant hydroalcoolique,
- d'un polyméthacrylate, notamment l'EUDRAGIT NE 30 D en mélange avec un dérivé cellulosique soluble, notamment de l'hydroxypropyl-méthylcellulose et un plastifiant et/ou un agent soluble diluant à propriétés liantes,
- d'EUDRAGIT E 100 seul.

Grâce à l'enrobage, conforme à l'invention, des microcristaux de substance active, cet enrobage comprenant notamment en combinaison un polymère soluble et un polymère insoluble, le comprimé définitif est caractérisé par le fait
- d'une part, qu'en milieu acide, dont le pH est inférieur à 5, la quantité de principe actif qui est dissoute, après la désagrégation du comprimé, en 5 à 20 minutes à partir des microcristaux enrobés est égale à au moins 80% et de préférence à au moins 100% de la quantité de principe actif qui est dissoute dans le même délai après désagrégation à partir d'un comprimé permettant la libération immédiate du principe actif constitué par des microcristaux mais dans lequel lesdits microcristaux ne sont pas enrobés et,
- d'autre part, que le principe actif ne se dissout pas de façon significative après un séjour d'une durée inférieure à 5 minutes dans un milieu dont les conditions de pH sont proches de celles de la salive, c'est-à-dire de 7,0±0,5, assurant ainsi un masquage du goût satisfaisant.

Lorsque la quantité de principe actif qui est dissoute après la désagrégation du comprimé en 5 à 20 minutes à partir des microcristaux enrobés est égale à au moins 100% de la quantité de principe actif qui est dissoute dans le même délai après désagrégation à partir d'un comprimé permettant la libération immédiate du principe actif constitué par des microcristaux mais dans lesquels lesdits microcristaux ne sont pas enrobés, la biodisponibilité de la substance active est au moins équivalente à celle du même principe actif obtenue à partir du susdit comprimé dans lequel lesdits microcristaux ne sont pas enrobés.

Pour la fabrication du comprimé, on prépare tout d'abord un mélange de l'excipient et des microcristaux enrobés et on procède à une homogénéisation dans un mélangeur à sec.

De préférence, on fait comporter à ce mélange un édulcorant, un arôme et un lubrifiant.

L'édulcorant peut être choisi dans le groupe comprenant l'aspartame et le saccharinate de sodium, et le lubrifiant dans le groupe comprenant le stéarate de magnésium, le stéarylfumarate de sodium, l'acide stéarique et le polyéthylèneglycol 6000.

Le mélange est ensuite soumis à une force de compression suffisante pour conférer au comprimé résultant une dureté suffisante pour qu'il puisse être manipulé et conditionné industriellement, puis transporté et manipulé par le patient sans précautions particulières; à titre indicatif, on signale que des duretés répondant à ces conditions sont généralement comprises entre 20 et 70 Newtons.

Les comprimés conformes à l'invention présentent, par rapport aux comprimés du genre en question qui existent déjà, à la fois une amélioration de la vitesse de mise à disposition du principe actif dans l'organisme et une amélioration de la palatabilité.

### EXEMPLE 1

### Comprimé multiparticulaire de paracétamol dosé à 500 mg.

La composition du susdit comprimé résulte du tableau I ci-après.

**TABLEAU I**

| Constituants | Formule centésimale |
|---|---|
| Paracétamol enrobé | 39,2 |
| Mannitol pour compression directe | 36,7 |
| Mannitol poudre cristalline | 12,3 |
| Crospovidone | 8,6 |
| Aspartame | 2,7 |
| Arôme cassis | 0,4 |
| Stéarate de magnésium | 0,1 |
| Total | 100,0 % |

Ce comprimé est préparé comme indiqué ci-après.

On introduit les microcristaux de paracétamol dans une installation à lit fluidisé et on pulvérise sur les microcristaux une dispersion dans l'éthanol d'EUDRAGIT E 100, d'EUDRAGIT NE 30 D et de silice colloïdale de façon à obtenir des microcristaux enrobés avec 10% de polymère.

On tamise tous les excipients et on homogénéise le mélange comprenant le paracétamol enrobé et les excipients dans un mélangeur à sec.

On procède à la répartition et à la mise en forme sur comprimeuse équipée de poinçons de diamètre 17 mm.

La force de compression est réglée de manière à obtenir des comprimés d'une dureté de 40±10 Newtons.

Le temps de désagrégration dans la bouche des comprimés ainsi obtenus est inférieur à 40 secondes.

Ce temps correspond à la durée qui sépare, d'une part, le moment de la mise en place du comprimé dans la bouche au contact de la salive et, d'autre part, le moment de la déglutition de la suspension résultant de la désagrégation du comprimé au contact de la salive.

Le temps de désagrégation inférieur à 40 secondes indiqué plus haut est la moyenne des valeurs notées par un groupe représentatif de sujets sains.

L'étude pharmacocinétique à laquelle a été soumis le susdit comprimé montre que la biodisponibilité de la substance active, à savoir le paracétamol, n'est pas significativement différente de celle qui est observée après administration d'un comprimé du commerce à base de paracétamol. Le masquage du goût du paracétamol par l'enrobage comporté par le comprimé conforme à l'invention n'entraîne donc pas de retard à l'absorption, celle-ci étant même légèrement plus rapide puisque le maximum est atteint en 0,5 heure en moyenne alors qu'il n'est atteint qu'après 0,88 heure en moyenne dans le cas du comprimé de référence commercialisé.

Les valeurs enregistrées en rapport avec les propriétés du comprimé selon l'invention et celles d'un comprimé à base de paracétamol déjà commercialisé à libération immédiate du principe actif non préalablement enrobé, sont réunies dans le tableau II ci-après.

**TABLEAU II**

| Paramètres | Comprimé dosé à 500 mg de paracétamol selon l'invention | Comprimé du commerce dosé à 500 mg de paracétamol |
|---|---|---|
| Lag t (h) | 0,17 | 0,17 |
| tmax (h) | 0,50 | 0,88 |
| Cmax (µg/ml) | 6,28 ± 1,61 | 6,26 ± 2,37 |
| AUC0-t (µg.h/ml) | 18,59 ± 3,44 | 18,10 ± 3,40 |
| AUCinf (µg.h/ml) | 19,68 ± 3,85 | 19,24 ± 3,79 |

Dans ce tableau,
- lag t désigne le temps, exprimé en heures, écoulé entre l'administration du médicament et la détection du principe actif dans le sang du sujet,
- tmax désigne le temps, exprimé en heures, au bout duquel la concentration sérique en principe actif atteint un maximum,
- Cmax (µg/ml) désigne la valeur de la concentration maximale en principe actif atteinte à la valeur tmax; cette concentration s'exprime en µg de principe actif par ml de sérum,
- AUC0-t désigne la surface sous courbe des concentrations sériques du principe actif en fonction du temps jusqu'au dernier prélèvement quantifié, et
- AUCinf désigne la surface sous courbe des concentrations sériques du principe actif en fonction du temps extrapolé à l'infini.

L'examen des résultats réunis dans le tableau II montre que l'enrobage utilisé pour masquer le goût du principe actif n'entraîne pas de modification de la biodisponibilité du principe actif et aucun retard d'absorption, ce qui est un résultat déterminant dans l'administration de certains principes actifs dont une action rapide est souhaitée, en particulier les antalgiques.

### EXEMPLE 2

### Comprimé multiparticulaire de lopéramide dosé à 2 mg.

La composition du susdit comprimé résulte du tableau III ci-après.

**TABLEAU III**

| Constituants | Formule centésimale |
|---|---|
| Lopéramide enrobé | 15,1 |
| Mannitol pour compression directe | 56,6 |
| Mannitol poudre cristalline | 18,8 |
| Crospovidone | 5,0 |
| Aspartame | 3,0 |
| Arôme menthe | 0,7 |
| Stéarate de magnésium | 0,8 |
| Total | 100,0 % |

Ce comprimé est préparé de la manière indiquée à l'exemple 1.

Le principe actif se présente sous forme de microcristaux dont la taille moyenne est inférieure à 20 micromètres; il est donc procédé à une fixation, à l'aide d'une solution d'hydroxypropyl-méthylcellulose, sur des supports neutres constitués d'un polyol et dont la taille moyenne est de l'ordre de 60 micromètres.

Les supports neutres comportant les microcristaux de lopéramide sont ensuite enrobés de 20% d'un mélange d'EUDRAGIT NE 30 D et d'EUDRAGIT E 100.

Ce comprimé présente une dureté de 30±5 Newtons.

Il se désintègre dans la bouche en 20 secondes.

### EXEMPLE 3

### Comprimé de kétoprofène dosé à 12,5 mg.

La composition du susdit comprimé résulte du tableau IV ci-après.

**TABLEAU IV**

| Constituants | Formule centésimale |
|---|---|
| Kétoprofène enrobé | 6,7 |
| Mannitol pour compression directe | 63,3 |
| Mannitol poudre cristalline | 22,2 |
| Crospovidone | 5,0 |
| Aspartame | 1,3 |
| Arôme menthe | 0,7 |
| Stéarate de magnésium | 0,8 |
| Total | 100,0 % |

Il est préparé de la manière indiquée à l'exemple 2.

Le principe actif se présente sous forme de microcristaux dont la taille moyenne est inférieure à 20 micromètres; il est donc procédé à une fixation, à l'aide d'une solution d'hydroxypropyl-méthylcellulose, sur des supports neutres constitués d'un polyol et dont la taille moyenne est de l'ordre de 60 micromètres.

Les supports neutres comportant les microcristaux de kétoprofène constitutifs du susdit comprimé sont ensuite enrobés de 20% d'un mélange d'EUDRAGIT NE 30 D, d'hydroxypropyl-méthylcellulose et de polyéthylène-glycol 6000.

Il présente une dureté de 35±5 Newtons.

Il se désintègre dans la bouche en 20 secondes.

### EXEMPLE 4

### Comprimé multiparticulaire d'acide acétylsalicylique dosé à 325 mg.

La composition du susdit comprimé résulte du tableau V ci-après.

**TABLEAU V**

| Constituants | Formule centésimale |
|---|---|
| Acide acétylsalicylique enrobé | 37,4 |
| Mannitol pour compression directe | 38,0 |
| Mannitol poudre cristalline | 12,6 |
| Crospovidone | 8,6 |
| Aspartame | 2,9 |
| Stéarylfumarate de sodium | 0,5 |
| Total | 100,0 % |

Ce comprimé est préparé de la manière indiquée à l'exemple 1.

Les microcristaux d'acide acétylsalicylique constitutifs du susdit comprimé sont enrobés de 4,5% d'éthylcellulose N7 et de polyéthylèneglycol 6000.

Ce comprimé présente une dureté de 50±15 Newtons.

Il se désintègre dans la bouche en 20 secondes.

### EXEMPLE 5

### Comprimé multiparticulaire d'ibuprofène dosé à 100 mg.

La composition du susdit comprimé résulte du tableau VI ci-après.

**TABLEAU VI**

| Constituants | Formule centésimale |
|---|---|
| Ibuprofène enrobé | 14,4 |
| Mannitol pour compression directe | 52,5 |
| Mannitol poudre cristalline | 17,5 |
| Crospovidone | 10,0 |
| Aspartame | 3,7 |
| Arôme cerise | 0,9 |
| Stéarate de magnésium | 1,0 |
| Total | 100,0 % |

Ce comprimé est préparé de la manière indiquée à l'exemple 1.

On granule les microcristaux d'ibuprofène dont la taille moyenne est voisine de 30 micromètres par un procédé classique de granulation par voie humide. La particule résultant est ensuite enrobée de 20% d'un mélange d'éthylcellulose N7 et de polyéthylèneglycol 6000.

Les cristaux d'ibuprofène enrobés résultant sont mélangés à l'excipient, puis soumis à la compression.

Le comprimé résultant présente une dureté de 40±10 Newtons.

Il se désintègre dans la bouche en 30 secondes.

## Revendications

1. Comprimé multiparticulaire perfectionné se désintègrant dans la bouche en moins de 40 secondes et comprenant, d'une part, une substance active sous forme de microcristaux enrobés et, d'autre part, un excipient, caractérisé par le fait
- que l'excipient comprend au moins un agent désintégrant et au moins un agent soluble diluant à propriétés liantes constitué par un polyol de moins de 13 atomes de carbone se présentant soit sous la forme du produit directement compressible dont le diamètre moyen des particules est de 100 à 500 micromètres, soit sous la forme d'une poudre dont le diamètre moyen des particules est inférieur à 100 micromètres, ce polyol étant de préférence choisi dans le groupe comprenant le mannitol, le xylitol, le sorbitol et le maltitol, étant entendu que le sorbitol ne peut être utilisé seul et que, dans le cas où l'agent soluble diluant à propriétés liantes est unique, il est utilisé sous la forme du produit directement compressible alors que, dans le cas où il y a au moins deux agents solubles diluants à propriétés liantes, l'un est présent sous la forme directement compressible et l'autre sous la forme poudre, le polyol pouvant alors être le même, les proportions de polyol directement compressible et de polyol poudre étant de 99/1 à 50/50, de préférence de 80/20 à 50/50 et
- que l'enrobage des microcristaux de substance active comprend au moins un agent d'enrobage choisi en fonction des caractéristiques physico-chimiques de la substance active dans le groupe comprenant les polyméthacrylates, les polymères cellulosiques, notamment les éthylcelluloses, les hydroxypropyl-méthylcelluloses, les hydroxypropyl-celluloses et les acétophthalates de cellulose et les combinaisons de ces polymères l'un avec l'autre éventuellement associés à des plastifiants ou des agents solubles, notamment les polyols.

2. Comprimé multiparticulaire perfectionné se désintègrant dans la bouche en moins de 40 secondes et comprenant, d'une part, une substance active sous forme de microcristaux enrobés et, d'autre part, un excipient, caractérisé par le fait
- d'une part, qu'en milieu acide, dont le pH est inférieur à 5, la quantité de principe actif qui est dissoute après la désagrégation du comprimé en 5 à 20 minutes à partir des microcristaux enrobés est égale à au moins 80% et de préférence à au moins 100% de la quantité de principe actif qui est dissoute dans le même délai après désagrégation à partir d'un comprimé permettant la libération immédiate du principe actif constitué par des microcristaux mais dans lequel lesdits microcristaux ne sont pas enrobés et,
- d'autre part, que le principe actif ne se dissout pas de façon significative après un séjour d'une durée inférieure à 5 minutes dans un milieu dont les conditions de pH sont proches de celles de la salive, c'est-à-dire de 7,0±0,5, assurant ainsi un masquage du goût satisfaisant.

3. Comprimé multiparticulaire perfectionné selon la revendication 2, caractérisé par le fait que la biodisponibilité de la substance active est au moins équivalente à celle du même principe actif obtenue à partir d'un comprimé permettant la libération immédiate du principe actif constitué par des microcristaux mais dans lequel lesdits microcristaux ne sont pas enrobés.

4. Comprimé multiparticulaire perfectionné selon l'une des revendications 1 à 3, caractérisé par le fait que la substance active est choisie dans le groupe comprenant les antalgiques, les antipyrétiques, les antidiarrhéiques, les antispasmodiques, les régulateurs de la motricité digestive et les anti-inflammatoires et plus particulièrement dans celui comprenant le paracétamol, l'ibuprofène, l'aspirine, le kétoprofène, le lopéramide.
